# EUROPEAN PATENT SPECIFICATION

(11) **EP 2 204 653 B1**
(45) Date of publication and mention of the grant of the patent: **17.09.2014**
(21) Application number: 07872127.1
(22) Date of filing: 12.10.2007
(51) Int. Cl.: G01N 33/543, G01N 33/569, C12Q 1/04

(54) **DIAGNOSTICUM FOR DETERMINING THE PRESENCE OF TOTAL ENDOTOXIN (LIPOPOLYSACCHARIDE-LPS) OF GRAM-NEGATIVE BACTERIA AND ALSO THE GENUS AND TYPE OF GRAM-NEGATIVE BACTERIA PRODUCING ENDOTOXIN, METHOD FOR PRODUCING SAID DIAGNOSTICUM AND A SET**
DIAGNOSTIKUM ZUR BESTIMMUNG DES VORLIEGENS VON GESAMT-ENDOTOXIN (LIPOPOLYSACCHARID LPS) GRAMNEGATIVER BAKTERIEN SOWIE DER GATTUNG UND ART ENDOTOXIN PRODUZIERENDER GRAMNEGATIVER BAKTERIEN, VERFAHREN ZUR HERSTELLUNG DES DIAGNOSTIKUMS UND SET
AGENT DE DIAGNOSTIC POUR DÉTERMINER LA PRÉSENCE D'UNE ENDOTOXINE GÉNÉRALE (LIPOLYSACCHARIDE LPS) DE BACTÉRIES GRAM NÉGATIF ET DU GENRE ET TYPE DE BACTÉRIES GRAM NÉGATIF PRODUISANT DES ENDOTOXINES, PROCÉDÉ DE FABRICATION DE CET AGENT DE DIAGNOSTIC ET KIT CORRESPONDANT

(43) Date of publication of application: 07.07.2010
(73) Proprietor: Gosudarstvennoe Uchrezhdenie Nauchny Tsentr Serdechno-Sosudistoi Khirurgii IM. A.N. Bakuleva Rossiiskoi Academii Meditsinskikh Nauk, Moscow 119991 (RU); Obschestvo S Ogranichennoi Otvetstvennostju "Nauchno-Proizvodstvennaya Firma 'ROKHAT', Moscow 101000 (RU); Bokeriya, Leonid Antonovich, Moscow 117049 (RU); Niyazmatov, Agzamdzhan Akhtamovich, Moscow 121615 (RU)
(72) Inventor: BOKERIYA, Leonid Antonovich, Moscow 117049 (RU); NIYAZMATOV, Agzamdzhan Akhtamovich, Moscow 121615 (RU)
(74) Representative: HOFFMANN EITLE
(86) International application number: PCT/RU2007/000558
(87) International publication number: WO 2009/048350

(56) References cited:
- RU-C1- 2 189 253
- RU-C2- 2 266 133
- RU-C2- 2 266 134
- RU-C2- 2 266 135
- RU-C2- 2 266 136
- RU-C2- 2 266 137
- US-A1- 2004 142 398

## Description

### FIELD OF INVENTION

The invention relates to medicine, veterinary science, and the food industry and can be used for endotoxin determination.

### BACKGROUND OF THE INVENTION

There is known a diagnosticum for identifying legionellas in a clinical material and a method for producing the diagnosticum comprising a carrier in the form of an atraquinone blue colored polystyrene latex with activated amino groups and a specific rabbit immunoglobulin to the surface protein antigen of L. pneumophila; the diagnosticum is a 0.5% suspension of the latex particles covalent-bound to the specific rabbit immunoglobulin (RU 2129279 C1, 20.04.1999). The diagnosticum does not permit to determine for a short time the presence of the total LPS of gram-negative bacteria and to identify the genus and type of endotoxin-prodicing gram-negative bacteria, i.e., to carry out the step-by-step instant diagnosis, starting with the detection of the presence of endotoxins of gram-negative bacteria to the detection of the endotoxin of the given genus and type of gram-negative bacteria.

There is known a kit for detecting antibodies to the hepatitis C virus; the kit comprising a combination of structural and non-structural antigens to the hepatitis C virus containing immunoactive epitopes immobilized on the surface of a solid-phase carrier and also comprising polypeptides as antigens (RU 2262704 C1, 20.10.2005). The kit does not permit to carry out an instant diagnosis of the presence of the total LPS of gram-negative bacteria and to determine for a short time the genus and type of endotoxin-prodicing gram-negative bacteria.

There is known a method for producing a diagnosticum for determining antigens to the hepatitis B virus, wherein a styrene copolymer latex is sensitized with antibodies in an 0.1 M glycine buffer of pH 7.0 and then is incubated at 37°C for 2 to 8 hours (N.A. Chaika, E.N. Gorbachev "Primenenie reaktsii agglyutinatsii sensibilizirovannogo lateksa dlya diagnostiki virusnykh infektsii [Use of an agglutination reaction of sensitized latex for diagnosis of viral infections], Voprosy virusologii, 1985, no. 5, pp 516-523). The method makes it possible to produce for a short time a diagnosticum for detecting the hepatitis B virus at a concentration thereof of 1 µg/ml. The drawbacks of the diagnosticum are the low sensitivity, specificity (68.02±0.88), and a long time of obtaining the results (20 min); the diagnosticum does not permit to carry out instantly detecting the presence of the total LPS of gram-negative bacteria and also determining for a short time the genus and type of endotoxin-producing gram-negative bacteria.

There is known a method for producing a diagnosticum for determining endotoxins of gram-negative bacteria comprising the use of an antibody diagnosticum (US 5316911 A1, 31.05.1994). The method makes it possible to produce a highly sensitive diagnosticum; however the process is multi-step and laborious and the method does not provide instantly determining the presence of the total LPS of gram-negative bacteria and also identifying the genus and type of endotoxin-producing gram-negative bacteria.

The closest art to the present invention is a method for producing a diagnosticum for determining endotoxins of gram-negative bacteria; the method comprising the use of antibody diagnosticums, wherein a core-shell type latex is used as a carrier, where the core is a polystyrene latex and the shell is a styrene and zinc methacrylate copolymer latex (RU 2266137 C2, 27.01.2005). However, the method does not permit instantly determining the presence of the total LPS of gram-negative bacteria and also the genus and type of endotoxin-producing gram-negative bacteria.

RU 2 189 253 C1 relates to the development of a diagnosticum designated for identification of *E*. *coli* 0157:H7 for epidemiological inspection and diagnosis of hemorrhagic colitis. The diagnosticum comprises a carrier and sensitine. As a carrier the diagnosticum comprises polystyrene latex stained by lipid-soluble blue antraquine dye with activated amino-groups having particles size 0.502 mcm, variation coefficient is 4.1-4.5%, dispersion coefficient is 1.003-1.005, concentration of amino-groups is 2.3 x (10-3 - 10-4) mole/g and as sensitine the diagnosticum comprises protein A and rabbit immunoglobulins showing specificity raised to polysaccharide fraction LPS 0157 and protein flagellar antigen H7. For preparing the indicated diagnosticum 1-% suspension of preliminary washed out particles used as a carrier of polystyrene latex is incubated with protein A in concentration 55 mcg/ml as a sensitine in their equal ratio 1:1 and then washed out particles are incubated with rabbit specific immunoglobulins raised to polysaccharide fraction of lipopolysaccharide 0157, protein flagellar antigen H7 in concentration 180 mcg and 210 mcg, respectively, in their volume ratio 1:1. After incubation latex particles are treated with glycine buffer at pH 8.2 and incubated repeatedly. The prepared diagnosticum presents 0.5-% suspension of latex particles that are bound covalently with indicated rabbit specific immunoglobulins. The diagnosticum can be used for laboratory diagnosis of hemorrhagic colitis and for identification of *E*. *coli* 0157: H7 in foodstuffs.

US 2004/142 398 A1 refers to a method for performing an immunoassay. The method is used for detecting extracellular polysaccharide (EPS) and/or lipopolysaccharide (LPS) producing microorganisms. The EPS and/or LPS can be extracted from a sample with cetyltrimethylammonium bromide (CTAB) to produce molecular aggregates which are then preferentially bound to colored polystyrene latex particles over other components in the sample, and the bound EPS and/or LPS detected using a lateral flow immunoassay apparatus which has immobilized thereon antibodies specific for the EPS and/or LPS. The method can also be used to detect particular viruses, for example viruses of the potyviridae or tobamoviridae group.

RU 2 266 137 C2 describes the manufacturing of corn-shell-type latex, wherein corn represents polystyrene latex and shell represents styrene-zinc methacrylate copolymer latex in repeating unit mass ratio of 1:0.8-1:0.4. Then latex is sensitized with monoclonal antibodies of IgG2a and IgG2b fractions in 0.01 M glycine buffer at pH 8.2-8.6 without preliminary conditioning followed by incubation primarily at 36-38 °C for 120-180 min and further at 2-8 °C for 1-20 hours. The obtained diagnosticum has a specificity of 99+-0.89, sensibility up to 10 pg/ml and makes it possible to obtain investigation results for 10 min or less.

RU 2 266 133 C2 relates to a method that includes manufacturing of corn-shell-type latex, wherein corn represents polystyrene latex and shell represents styrene-zinc methacrylate copolymer latex in repeating unit mass ratio of 1:0.8-1:0.4. Then latex is sensitized with monoclonal antibodies of IgG2a and IgG2b fractions in 0.01 M glycine buffer at pH 7.2-8.8 without preliminary conditioning followed by incubation primarily at 37-37.5 °C for 60-90 min and further at 2-8 °C for 22 hours. The obtained diagnosticum has a specificity of 98+-0.2, sensibility up to 10 pg/ml and makes it possible to obtain investigation results for 10 min or less.

RU 2 266 134 C2 discloses a method that includes manufacturing of corn-shell-type latex, wherein corn represents polystyrene latex and shell represents styrene-zinc methacrylate copolymer latex in repeating unit mass ratio of 1:0.8-1:0.4. Then latex is sensitized with monoclonal antibodies of IgG2a and IgG2b fractions in 0.01 M glycine buffer at pH 7.2-8.8 without preliminary conditioning followed by incubation primarily at 3.5-37 °C for 100-120 min and further at 6-8 °C for 20 hours. The obtained diagnosticum has a specificity of 98+-1.0, sensibility up to 10 pg/ml and makes it possible to obtain investigation results for 10 min or less.

RU 2 266 135 C2 refers to a method that includes manufacturing of corn-shell-type latex, wherein corn represents polystyrene latex and shell represents styrene-zinc methacrylate copolymer latex in repeating unit mass ratio of 1:0.8-1:0.4. Then latex is sensitized with monoclonal antibodies of IgG2a and IgG2b fractions in 0.01 M glycine buffer at pH 7.2-8.8 without preliminary conditioning followed by incubation primarily at 37-39 °C for 90-159 min and further at 10 °C for 10 hours. The obtained diagnosticum has a specificity of 99+-0.3, sensibility up to 10 pg/ml and makes it possible to obtain investigation results for 10 min or less.

RU 2 266 136 C2 describes a method which includes manufacturing of corn-shell-type latex, wherein corn represents polystyrene latex and shell represents styrene-zinc methacrylate copolymer latex in repeating unit mass ratio of 1:0.8-1:0.4. Then latex is sensitized with monoclonal antibodies of lgG2a and lgG2b fractions in 0.01 M glycine buffer at pH 7.2-8.8 without preliminary conditioning followed by incubation-primarily at 37 °C for 130-150 min and further at 8 °C for 18 hours. The obtained diagnosticum has a specificity of 97+- 0.6, sensibility up to 10 pg/ml and makes it possible to obtain investigation results for 10 min or less.

### SUMMARY OF THE INVENTION

The problem to be solved by the present invention is to produce a diagnosticum that makes it possible to carry out with a high sensitivity and specificity an abacterial (without bacterial inoculation) instant diagnosis of the presence of the total LPS of gram-negative bacteria and determining the genus and type of endotoxin-producing gram-negative bacteria, to reduce the time of producing the diagnosticum, and also to provide a step-by-step instant diagnosis starting with the detection of the presence of the total LPS of gram-negative bacteria to the detection of the endotoxin of the given genus and type of a gram-negative bacterium for no more than 10 min.

Thus, one of the subject matters of the invention is a diagnosticum for determining the presence of the total LPS of gram-negative bacteria and also the genus and type of endotoxin-producing gram-negative bacteria obtainable by a method according to any one of claims 1-8, having a sensitivity of up to 4pg/ml; the diagnosticum consisting of:
(a) a carrier in the form of a polystyrene latex (polystyrene chemical microspheres - PCM) with mono- or polyclonal antibodies sensitized thereon, said polystyrene latex carrier being represented by core-shell type latex particles, in which the core is the polystyrene latex and the shell is the styrene and zinc methacrylate copolymer latex at a mass unit ratio ranging from 1:0.8 to 1:0.4;
(b) mono- or polyclonal antibodies, in particular of an IgG2a and IgG2b or IgG3 and IgG2a or IgG1 and IgG2a-k or IgG1 and IgG2a subclass, with which the latex particles are sensitized.

The diagnosticum exhibits a high sensitivity of up to 4 pg/ml and specificity of up to 98.7-99.5%.

One more subject matter of the invention is a method for producing a diagnosticum for determining the presence of the total LPS of gram-negative bacteria and also the genus and type of endotoxin-producing gram-negative bacteria; the method comprising the steps of:
(a) providing a core-shell type latex, e.g., in a reactor, which is preliminarily blasted with nitrogen for about 30 min, then is loaded with about 10% of styrene, about 0.1% of potassium persulfate, and 89.9% of water; the mixture is thermostated and stirred at about 70°C for 7 h until full conversion of the monomer;
(b) adding to the latex of step (a) about 0.2% of styrene, about 0.1% of zinc methacrylate, and about 0.05% of potassium persulfate;
(c) stirring the mixture of step (b) until dissolution of all components;
(d) thermostating at about 70°C for about 2 h until latex particles are formed; and
(e) separating the latex particles of step (d) (the particles of latex thus obtained consist of two parts: the internal core that is polystyrene and the outer shell (about 0.2 to 20% of the particle mass) that is a static copolymer of styrene and zinc methacrylate at a mass unit ratio of about 1:0.8 to 1:0.4; the mass unit ratio of zinc methacrylate and styrene both in the core and in the shell is about 1-12:100);
(f) sensitizing the latex particles with mono- or polyclonal antibodies, in particular of an IgG2a, IgG2b, IgG3, IgG1, and IgG2a-k fraction (commercially available from manufacturers, in particular, Biogenesis (UK), Chemicon (USA), and others) in an 0.01 M glycine buffer of pH 7.0 to 8.8 until complete binding (a step of preliminary holding of the latex in the buffer is ruled out);
(g) a first incubation of the product of step (f) at about 38-40°C for no more than 50-70 min;
(h) a second incubation of the product of step (f) at about 36-38°C for no more than 50-70 min;
(i) a third (final) incubation at about 4-8°C for about 2-4 h.

The optimum time of sensitizing along with the incubation process performed under mild conditions yields an unexpected result, i.e., enhancement of the sensitivity and specificity of the diagnosticum, reduction of the time of production thereof, and reduction of the time of determining the presence of the total LPS of gram-negative bacteria and the genus and types of endotoxins of gram-negative bacteria.

The method of the invention is carried out as described in claims 1-8.

One more subject matter of the invention is a kit as described in claim 10.

### EMBODIMENT OF THE INVENTION

The inventors have established that producing a diagnosticum for determining the presence of the total LPS of gram-negative bacteria and also the genus and type of endotoxins of gram-negative bacteria, in particular Serratia, Klebsiella, Pseudomonas, Enterobacter, with high sensitivity and specificity depends on: pH of the buffer solution, wherein sensitizing is performed; the initial temperature and duration of the incubation; and the duration of the first, second, and third incubations.

Table 1 shows data on the dependence of the diagnosticum sensitivity on pH of the glycine buffer at a temperature of the first incubation of 39°C(for 60 min), at a temperature of the second incubation of 37°C (for 60 min), and at a temperature of the third incubation of 5°C (for 3 h).

**Table 1**

| Item | Number of tests | pH of glycine buffer | Diagnosticum sensitivity | |
|---|---|---|---|---|
| | | | Microbial bodies/ml | pg/ml |
| 1 | 400 | 7.0 | 250,000 | 250 |
| 2 | 400 | 7.2 | 125,000 | 125 |
| 3 | 400 | 7.6 | 60,000 | 60 |
| 4 | 400 | 8.0 | 30,000 | 30 |
| 5 | 400 | 8.4 | 15,000 | 15 |
| 6 | 400 | 8.6 | 7,500 | 7.5 |

Table 2 shows data on the dependence of the diagnosticum sensitivity on the temperature of the first incubation: at the buffer pH 8.6, the duration of the first incubation 60 min, the duration of the second incubation 60 min at 37°C, and the temperature of the third incubation 3 h at 5°C.

**Table 2**

| Item | Number of tests | Temperature of the 1^{st} incubation | Diagnosticum sensitivity | |
|---|---|---|---|---|
| | | | Microbial bodies/ml | pg/ml |
| 1 | 200 | 38°C | 6,000 | 7.5 |
| 2 | 200 | 38.5°C | 8,000 | 15 |
| 3 | 200 | 39°C | 8,000 | 30 |
| 4 | 200 | 40°C | 8,000 | 60 |

Table 3 shows data on the dependence of the diagnosticum sensitivity on the duration of the first incubation: at the buffer pH 8.6; the temperature of the first incubation 38.5°C, the temperature of the second incubation 37°C for 60 min, and the temperature of the third incubation 5 °C for 3 h.

**Table 3**

| Item | Number of tests | Time of the 1^{st} incubation, min | Diagnosticum sensitivity | |
|---|---|---|---|---|
| | | | Microbial bodies/ml | pg/ml |
| 1 | 120 | 50 | 2,000 | 30 |
| 2 | 120 | 55 | 2,000 | 15 |
| 3 | 120 | 60 | 1,000 | 7.5 |
| 4 | 120 | 65 | 1,000 | 7.5 |
| 5 | 120 | 70 | 1,000 | 7.5 |

Table 4 shows data on the dependence of the diagnosticum sensitivity on the temperature of the second incubation: pH 8.6; the temperature of the first incubation 38.5°C, the duration 60 min; the duration of the second incubation 60 min, the temperature and duration of the the third incubation 5°C and 3 h.

**Table 4**

| Item | Number of tests | Temperature of the 2^{nd} incubation | Diagnosticum sensitivity | |
|---|---|---|---|---|
| | | | Microbial bodies/ml | pg/ml |
| 1 | 160 | 36°C | 10,000 | 30 |
| 2 | 160 | 36.5°C | 5,000 | 15 |
| 3 | 160 | 37°C | 1,000 | 7.5 |
| 4 | 160 | 38°C | 1,000 | 7.5 |
| 5 | 160 | 38.5°C | 1,000 | 7.5 |

Table 5 shows data on the dependence of the diagnosticum sensitivity on the duration of the second incubation: at the buffer pH 8.6; the duration and temperature of the first incubation 60 min and 38.5°C, the temperature of the second incubation 37°C, and the temperature and duration of the third incubation 5°C and 3 h.

**Table 5**

| Item | Number of tests | Temperature of the 2^{nd} incubation (h) | Diagnosticum sensitivity | |
|---|---|---|---|---|
| | | | Microbial bodies/ml | pg/ml |
| 1 | 100 | 50 | 5,000 | 30 |
| 2 | 100 | 55 | 1,000 | 15 |
| 3 | 100 | 60 | 1,000 | 7.5 |
| 4 | 100 | 65 | 1,000 | 7.5 |
| 5 | 100 | 70 | 15,000 | 7.5 |

Table 6 shows data on the dependence of the diagnosticum sensitivity on the temperature of the third incubation: at the buffer pH 8.6; the temperature and duration of the first incubation 38.5°C and 60 min, the temperature and duration of the second incubation 37°C and 60 min, and the duration of the third incubation 3 h.

**Table 6**

| Item | Number of tests | Temperature of completion of incubation | Diagnosticum sensitivity | |
|---|---|---|---|---|
| | | | Microbial bodies/ml | pg/ml |
| 1 | 100 | 4°C | 5,000 | 15 |
| 2 | 100 | 5°C | 1,000 | 7.5 |
| 3 | 100 | 6°C | 1,000 | 7.5 |
| 4 | 100 | 7°C | 1,000 | 7.5 |
| 5 | 100 | 8°C | 1,000 | 7.5 |

Table 7 shows data on the dependence of the diagnosticum sensitivity on the duration of the third incubation: at the buffer pH 8.6; the temperature and duration of the first incubation 38.5°C and 60 min, the temperature and duration of the second incubation 37°C and 60 min, and the temperature of the third incubation 5°C.

**Table 7**

| Item | Number of tests | Time of completion of incubation, h | Diagnosticum sensitivity | |
|---|---|---|---|---|
| | | | Microbial bodies/ml | pg/ml |
| 1 | 100 | 1 | 15,000 | 30 |
| 2 | 100 | 2 | 8,000 | 30 |
| 3 | 100 | 3 | 5,000 | 15 |
| 4 | 100 | 4 | 8,000 | 15 |
| 5 | 100 | 5 | 15,000 | 15 |

The specificity and sensitivity of the obtained diagnosticum for determining the presence of the total LPS of gram-negative bacteria and also the genus and types of endotoxins of gram-negative bacteria were tested in the reaction of MAP-Endotox spp, MAP-Serratia spp, MAP-Klebsiella spp, MAP-Pseudomonas spp, and MAP-Enterobacter spp with the donor blood serum that contained no endotoxin, with the blood serum of patients with cardiovascular diseases; and with the blood serum of patients with pyoinflammatory diseases.

The use of the diagnostic kits of the invention makes it possible to detect for ten minutes the presence of endotoxins of gram-negative bacteria in the blood serum of postoperative patients with cardiovascular pathologies and with proinflammatory diseases. It makes it possible to verify the efficiency and advisability of the therapy, in particular, the antibiotic therapy.

The reaction was performed with the use of disposable perforated 6-lunule cardboard planchettes including positive (C+) and negative (C-) control. The reaction results were assessed by the extent of particles activation:
1. (4CA) - particle activation of the 4^{th} degree - large flakes on the transparent background (distinctly positive result - in the range of 500-124 pg/ml).
2. (3CA) - particle activation of the 3^{rd} degree of - flakes or large grains on mildly cloudy background (positive result - in the range of 125-30 pg/ml).
3. (2CA) - particle activation of the 2^{nd} degree - many large grains on cloudy background (indistinctly positive result - in the range of 29-7.5 pg/ml).
4. (1CA) Control - particle activation of the 1^{st} degree - absolutely cloudy background or sporadic grains (negative result).

Table 8 shows the testing results.

**Table 8**

| Serum under study | Number of tests | Result |
|---|---|---|
| Donors (none endotoxin) | 100 | 100% negative |
| Patients with carduovascular diseases (confirmed by bacterial inoculation for gram-negative bacteria of various ethiologies) | 110 | 99.5% positive |
| | | 0.5% negative |
| Patients with pyoinflammatory diseases (confirmed by bacterial inoculation for gram-negative bacteria of various ethiologies) | 30 | 99% positive |
| | | 1% negative |

The specificity of the obtained diagnosticum for detecting endotoxins is 99±0.9%; the sensitivity, up to 4 pg/ml. The time necessary for producing the diagnosticum for determining the endotoxin is reduced from several days to several hours.

The invention is illustrated by the following examples which do not restrict the scope thereof.

### Example 1

The patient S. was hospitalized with a diagnosed cardiac pathology of medium severity. On the day of admission of the patient, there were carried out tests of the patient's blood serum for the presence of endotoxins of gram-negative bacteria. Towards that purpose, 100 µl of the serum were placed into lunules of disposable planchettes and were added with 100 µl of the diagnosticum kit of "MAP-Endotox spp", "MAP-Pseudomonas spp", "MAP-Enterobacter spp", "MAP-Serratia spp", and "MAP-Klebsiella spp"; the reagents were mixed and incubated for 10 min in a thermostat at 37°C being gently shaken 3 times for 3 min. After thermostating, the following results were obtained on the planchettes: there was detected an endotoxin of gram-negative bacteria of a high concentration - 250 pg/ml and identified as endotoxin of the Klebsiela genus and K.pneumoniae type. The patient was recommended an appropriate antibiotic therapy; note that the bacterial inoculation showed a negative result. After the therapeutic treatment the patient was operated on and then again a test was carried out for the presence of endotoxins of gram-negative bacteria; the test revealed the same endotoxin at the concentration thereof being 125 pg/ml. The patient was prescribed with an antibiotic therapy effective against that type of bacteria. After the corresponding treatment, the endotoxin concentration was 20 pg/ml. Before the patient was discharged from the hospital, there were carried out tests for the presence of endotoxins of gram-negative bacteria in the patient. The result was negative. The postoperative period passed without complications.

### Example 2

The patient A. was hospitalized with a diagnosed cardiac pathology of medium severity. On the day of admission of the patient, there were carried out tests of the patient's blood serum for the presence of endotoxins of gram-negative bacteria. Towards that purpose, 100 µl of the serum were placed into lunules of disposable planchettes and were added with 100 µl of the diagnosticum kit of "MAP-Endotox spp", "MAP-Pseudomonas spp", "MAP-Enterobacter spp", "MAP-Serratia spp", and "MAP-Klebsiella spp"; the reagents were mixed and incubated for 10 min in a thermostat at 37°C being gently shaken 3 times for 3 min. After thermostating, the following results were obtained on the planchettes: endotoxin was not detected. The patient was operated on; after the operation there were again carried out tests for the presence of endotoxins of gram-negative bacteria. There was detected an endotoxin of gram negative bacteria of the genus Pseudomonas of the type P. aeruginosa, the concentration thereof being 250 pg/ml. The patient was prescribed with an antibiotic therapy effective against that type bacteria. After the corresponding treatment, the endotoxin concentration remained unchanged - 250 pg/ml and the patient was administered a new antibiotic therapy course. After the new course of the antibiotic therapy, the level of endotoxin in the patient was 15 pg/ml. Before the patient was discharged from the hospital, there was again carried out a test for the presence of gram-negative bacteria in the patient. The result was negative. The postoperative period passed without complications.

The knowledge of the presence or absence of endotoxin in patients before discharge from the hospital is important because the presence of endotoxin may lead in future to complications if the patient is not administered a necessary treatment.

Different levels of endotoxin in the MAP reaction were detected in the blood serum of patients under extracorporal dialysis and in the blood serum of donors.

The use of diagnostic kits of the invention in surgery will make it possible:
(a) to determine within 10 min the type of gram-negative bacteria in a patient so that to take adequate measures;
(b) to verify the efficiency of the antibiotic therapy and to correct it against the level of endotoxin;
(c) to detect latent and chronic forms of endotoxemia, since the traditional bacterial inoculation technique has a 35% specificity and reveals gram-negative bacteria in cases of clinically apparent symptoms in a patient;
(d) the MAP reaction using the inventive diagnostic kits is easy to handle and requires no specific techniques and room, since all needed for carrying out the MAP reaction is contained in a pack and no highly skilled personnel is required; the reaction may be carried out and analyzed by a person of an ordinary medical skill;
(e) the specificity of the inventive instant-diagnosis kits is 98.7-99.5% (see Table 9).

It should be noted once again that a bacterial inoculation used in a traditional clinical practice takes 14 days to accomplish it and the instant-diagnosis using the diagnostic kits of the invention makes it possible to reduce the time for diagnosing to 10 min.

It should be emphasized that the sensitivity of the diagnostic kits of the invention is up to 4 pg/ml of endotoxin (see Table 9).

The diagnostic kits of the invention for determining the presence of endotoxins of gram-negative bacteria may be widely used in surgical practice, extracorporal dialysis, and for other pathological states. Table 9 shows the specificity and sensitivity of the diagnostic kits for determining the presence of the total LPS of gram-negative bacteria and, without restricting the scope of invention, in model experiments with specific and non-specific antigens to heating-inactivated microbial cultures.

It is evident from Table 9 that the specificity of the diagnostic test-systems for determining the presence of the total LPS of gram-negative bacteria and, without restricting the scope of invention, specific and non-specific antigens to heating-inactivated microbial cultures is 98.7±99.8; the sensitivity is from 30 to 4 pg/ml.

Specificity and sensitivity of the diagnostic kits for determining total and genus and types of endotoxins of gram-negative bacteria in model experiments with specific and nonspecific antigens and heating-inactivated microbial cultures

### Diagnostic kits

**Table 9**

| Item | Antigens and cultures | **MAP-Pseudomonas spp** | **MAP-Pseudomonas aeruginosa** | **MAP-Pseudomonas cepacia** | **MAP-Pseudomonas fluorescens** | **MAP-Pseudomonas maltophilia** | **MAP-Pseudomonas stutzeri** |
|---|---|---|---|---|---|---|---|
| 1 | Staphylococci | - | - | - | - | - | - |
| 2 | Streptococci | - | - | - | - | - | - |
| 3 | Enterococci | - | - | - | - | - | - |
| 4 | Candida-type fungi | - | - | - | - | - | - |
| 5 | E-coli LPS | + | + | + | + | + | + |
| 6 | Mixture of cultures Pseudomonas aeru,cepa,fluor malto, stut. | + | + | + | + | + | + |
| 7 | Pseudo aeruginosa culture | + | + | - | - | - | - |
| 8 | Pseudo cepacia culture | + | - | + | - | - | - |
| 9 | Pseudo fluorescens culture | + | - | - | + | - | - |
| 10 | Pseudo maltophilia culture | + | - | - | - | + | - |
| 11 | Pseudo stutzeri culture | + | - | - | - | - | + |
| 12 | Mixture of cultures Entcrobacter cloa,acro,alpha | - | - | - | - | - | - |
| 13 | Enterobac cloacae culture | - | - | - | - | - | - |

| Item | Antigens and cultures | **MAP-Pseudomonas spp** | **MAP-Pseudomonas aeruginosa** | **MAP-Pseudomonas cepacia** | **MAP-Pseudomonas fluoresce** | **MAP-Pseudomonas maltophilia** | **MAP-Pseudomonas stutzeri** |
|---|---|---|---|---|---|---|---|
| 14 | Enterobac aerogenes culture | - | - | - | - | - | - |
| 15 | Enterobac alpha culture | - | - | - | - | - | - |
| 16 | Mixture of cultures Klebsiella, oxy,pneu | - | - | - | - | - | - |
| 17 | Kleb oxytoca culture | - | - | - | - | - | - |
| 18 | Kleb pneumoniae culture | - | - | - | - | - | - |
| 19 | Mixture of cultures Serratia marc, serra&enterobac | - | - | - | - | - | - |
| 20 | Serra marcescens culture | - | - | - | - | - | - |
| 21 | Serra scrratia&enterobacter culture | - | - | - | - | - | - |
| 22 | LPS USA from LAL-test | - | - | - | - | - | - |
| 23 | Positive control of the Bakoulev Sci.entific Center for Cardiovascular Surgery, initial LPS concentration 500 pg/ml | 15 pg/ml | 15 pg/ml | 15 pg/ml | 15 pg/ml | 15 pg/ml | 30 pg/ml |
| 24 | .Acinobacter culture | - | - | - | - | - | - |

| Item | Antigens and cultures | **MAP-Enterobacter spp** | **MAP-Enterobacter cloacae** | **MAP-Enterobacter aerogenesa** | **MAP-Enterobacter alpha** | **MAP-Klebsiella spp** | **MAP-Klebsiella oxytoca** |
|---|---|---|---|---|---|---|---|
| 25 | Staphylococci | - | - | - | - | - | - |
| 26 | Streptococci | - | - | - | - | - | - |
| 27 | Enterococci | - | - | - | - | - | - |
| 28 | Candida-type fungi | - | - | - | - | - | - |
| 29 | E-coli LPS | + | + | + | + | + | + |
| 30 | Mixture of cultures Pseudomonas aeru,cepa,fluor malto, stut. | - | - | - | - | - | - |
| 31 | Pseudo aeruginosa culture | - | - | - | - | - | - |
| 32 | Pseudo cepacia culture | - | - | - | - | - | - |
| 33 | Pseudo fluorescens culture | - | - | - | - | - | - |
| 34 | Pseudo maltophilia culture | - | - | - | - | - | - |
| 35 | Pseudo stutzeri culture | - | - | - | - | - | - |
| 36 | Mixture of cultures Enterobacter cloa,acro,alpha | + | + | + | + | - | - |
| 37 | Enterobac cloacae culture | + | + | - | - | - | - |

| Item | Antigens and cultures | **MAP-Enterobacter spp** | **MAP-Enterobacter cloacae** | **MAP-Enterobacter aerogenesa** | **MAP-Enterobacter alpha** | **MAP-Klebsiella spp** | **MAP-Klebsiella oxytoca** |
|---|---|---|---|---|---|---|---|
| 38 | Enterobac aerogenes culture | + | - | + | - | - | - |
| 39 | Enterobac alpha culture | + | - | - | + | - | - |
| 40 | Mixture of cultures Klebsiella, oxy,pneu | - | - | - | - | + | + |
| 41 | Kleb oxytoca culture | - | - | - | - | + | + |
| 42 | Kleb pneumoniae culture | - | - | - | - | + | - |
| 43 | Mixture of cultures Serratia marc, serra&enterobac | - | - | - | - | - | - |
| 44 | Serra marcescens culture | - | - | - | - | - | - |
| 45 | Serra serratia &enterobacter culture | + | - | + | - | - | - |
| 46 | LPS USA from LAL-test | - | - | - | - | - | - |
| 47 | Positive control of Bakoulev Scientific Center for Cardiovascular Surgery, initial LPS concentration 500 pg/ml | 15 pg/ml | 15 pg/ml | 30pg/ml | 30 pg/ml | 15 pg/ml | 15 pg/ml |
| 48 | Acinobacter culture | - | - | - | - | - | - |

| Item | Antigens and cultures | | **MAP-Klebsiella pneumoniae** | **MAP-Serratia spp** | **MAP-Serratia marcescens** | **MAP-Serratia & Enterobacter** | **MAP-Endotox spp** |
|---|---|---|---|---|---|---|---|
| 49 | Staphylococci | | - | - | - | - | - |
| 50 | Streptococci | | - | - | - | - | - |
| 51 | Enterococci | | - | - | - | - | - |
| 52 | Candida-type fungi | | - | - | - | - | - |
| 53 | E-coli LPS | | + | + | + | + | + |
| 54 | Mixture of cultures Pseudomonas aeru,cepa,fluor malto, stut. | | - | - | - | - | + |
| 55 | Pseudo aeruginosa culture | | - | - | - | - | + |
| 56 | Pseudo cepacia culture | | - | - | - | - | + |
| 57 | Pseudo fluorescens culture | | - | - | - | - | + |
| 58 | Pseudo maltophilia culture | | - | - | - | - | + |
| 59 | Pseudo stutzeri culture | | - | - | - | - | + |
| 60 | Mixture of cultures Enterobacter cloa,acro,alpha | | - | + | - | + | + |
| 61 | Enterobac cloacae culture | | - | - | - | - | + |

| Item | Antigens and cultures | | **MAP-Klebsiella pneumoniae** | **MAP-Serratia spp** | **MAP-Serratia marce-scens** | **MAP-Serratia &Entero bacter** | **MAP-Endotox spp** |
|---|---|---|---|---|---|---|---|
| 62 | Enterobac aerogenes culture | | - | + | - | + | + |
| 63 | Enterobac alpha culture | | - | - | - | - | + |
| 64 | Mixture of cultures Klebsiella, oxy, pneu | | + | - | - | - | + |
| 65 | Kleb oxytoca culture | | - | - | - | - | + |
| 66 | Kleb pneumoniae culture | | + | - | - | - | + |
| 67 | Mixture of cultures Serratia marc, serra&enterobac | | - | + | + | + | + |
| 68 | Serra marcescens culture | | - | + | + | - | + |
| 69 | Serra serratia &enterobacter culture | | - | + | - | + | + |
| 70 | LPS USA from LAL-test | | - | - | - | - | + |
| 71 | Positive control of the Bakoulev Scientific Center for Cardiovascular Surgery, initial LPS concentration 500 pg/ml | | 15 pg/ml | 15 pg/ml | 15 pg/ml | 30 pg/ml | 4 pg/ml |
| 72 | Acinobacter culture | | - | - | - | - | - |

## Claims

1. A method for producing a diagnosticum for determining the presence of the total LPS of gram-negative bacteria and also the genus and type of endotoxin-producing gram-negative bacteria comprising the steps of:
(a) providing.a core-shell type latex by mixing 10% of styrene, 0.1% of potassium persulfate, and 89.9% of water; the mixture is thermostated and stirred at 70°C for 7 h until full conversion of the monomer;
(b) adding to the latex of step (a) 0.2% of styrene, 0.1% of zinc methacrylate, and 0.05% of potassium persulfate;
(c) stirring the mixture of step (b) until dissolution of all components;
(d) thermostating at 70°C for 2 h until latex particles are formed; and
(e) separating the latex particles of step (d);
(f) sensitizing the latex particles with mono- or polyclonal antibodies, in particular IgG2a, IgG2b, IgG3, IgG1, and IgG2a-k in an 0.01 M glycine buffer of pH 7.0 to 8.8 until complete binding;
(g) a first incubation of the product of step (f) at 38-40°C for no more than 50-70 min;
(h) a second incubation of the product of step (f) at 36-38°C for no more than 50-70 min;
(i) a third (final) incubation at 4-8°C for 2-4 h with obtaining the diagnosticum.

2. A method according to claim 1, wherein the glycine buffer pH is 7.0 to 8.8, the duration and temperature of the first incubation are about 60 min and about 39°C, the duration and temperature of the second incubation are about 60 min and about 37°C and the duration and temperature of the third incubation are about 3 h and about 5°C.

3. A method according to claim 1, wherein the glycine buffer pH is about 8.6, the temperature of the first incubation is about 38-40°C, the duration is about 60 min; the temperature and duration of the second incubation are about 60 min and about 37°C; and the duration and temperature of the third incubation are about 3 h and about 5°C.

4. A method according to claim 1, wherein the glycine buffer pH is about 8.6, the duration and temperature of the first incubation are about 60 min and about 39°C, the duration and temperature of the second incubation are about 60 min and about 37°C; and the duration and temperature of the third incubation are about 3 h and about 5°C.

5. A method according to claim 1, wherein the glycine buffer pH is about 8.6, the duration and temperature of the first incubation are about 60 min and about 38.5°C, the duration and temperature of the second incubation are about 60 min and about 36-38°C; and the duration and temperature of the third incubation are about 3 h and about 5°C.

6. A method according to claim 1, wherein the glycine buffer pH is about 8.6, the duration and temperature of the first incubation are about 60 min and about 38.5°C, the duration and temperature of the second incubation are about 60 min and about 37°C; and the duration and temperature of the third incubation are about 3 h and about 5°C.

7. A method according to claim 1, wherein the glycine buffer pH is about 8.6, the duration and temperature of the first incubation are about 60 min and about 38.5°C, the duration and temperature of the second incubation are about 60 min and about 37°C; and the duration and temperature of the third incubation are about 3 h and about 4-8°C.

8. A method according to claim 1, wherein the glycine buffer pH is about 8.6, the duration and temperature of the first incubation are about 60 min and about 38.5°C, the duration and temperature of the second incubation are about 60 min and about 37°C; and the duration and temperature of the third incubation are about 2 to 4 h and about 5°C.

9. A diagnosticum for determining the presence of the total LPS of gram-negative bacteria and also the genus and type of endotoxin-producing gram-negative bacteria obtainable by the method according to any one of claims 1-8, having a sensitivity of up to 4 pg/ml and comprising:
(a) a carrier in the form of a polystyrene latex with mono- or polyclonal antibodies sensitized thereon, said polystyrene latex carrier being represented by core-shell type latex particles, in which the core is the polystyrene latex and the shell is the styrene and zinc methacrylate copolymer latex at a mass unit ratio ranging from 1:0.8 to 1:0.4;
(b) mono- or polyclonal antibodies, in particular of an IgG2a and IgG2b or IgG3 and IgG2a or IgG1 and IgG2a-k or IgG1 and IgG2a subclass, with which the latex particles are sensitized.

10. A kit for determining the presence in a biological material of gram-negative bacteria, the total LPS of gram-negative bacteria, and also LPS general for the genus and type of endotoxin-producing gram-negative bacteria comprising the diagnosticum according to claim 9 produced by a method according to claim 1, which comprises:
(a) a container, wherein a diagnosticum is placed comprising a carrier sensitized with mono- or polyclonal antibodies, in particular of an IgG2a and IgG2b subclass for determining the presence of LPS common for all gram-negative bacteria;
(b) a container, wherein a diagnosticum is placed comprising a carrier sensitized with mono- or polyclonal antibodies, in particular of an IgG3 and IgG2a subclass for determining LPS common for the genus of bacterium, in particular the genus Serratia;
(c) a container, wherein a diagnosticum is placed comprising a carrier sensitized with mono- or polyclonal antibodies, in particular of an IgG1 and IgG2a-k subclass for determining LPS common for the genus of bacterium, in particular the genus Klebsiella;
(d) a container, wherein a diagnosticum is placed comprising a carrier sensitized with mono- or polyclonal antibodies, in particular of an IgG1 and IgG2a subclass for determining LPS common for the genus of bacterium, in particular the genus Pseudomonas;
(e) a container, wherein a diagnosticum is placed comprising a carrier sensitized with mono- or polyclonal antibodies, in particular of an IgG1 and IgG2a subclass for determining LPS common for the genus of bacterium, in particular the genus Enterobacter;
(f) a container, wherein a diagnosticum is placed comprising a carrier sensitized with mono- or polyclonal antibodies, in particular of an IgG2a, IgG2b, IgG3, IgG1, and IgG2a-k subclass to various types of gram-negative bacteria, in particular, Serratia, Klebsiella, Pseudomonas, and Enterobacter;
(g) a pack and directions for use.

## Patentansprüche

1. Verfahren zur Herstellung eines Diagnostikums zur Bestimmung der Präsenz des gesamten LPS von gramnegativen Bakterien und auch des Genus und Typs von Endotoxin-produzierenden gramnegativen Bakterien, wobei das Verfahren die folgenden Schritte umfasst:
(a) Bereitstellen eines Kern-Schale-artigem Latex, durch Vermischen von 10% Styren, 0,1% Kaliumpersulfat und 89,9% Wasser; die Mischung wird bei 70°C für 7 Stunden bis zur vollen Umwandlung des Monomers mit einem Thermostat behandelt und gerührt;
(b) Hinzufügen von 0,2% Styren, 0,1% Zink-Methacrylat und 0,05% Kaliumpersulfat zu dem Latex von Schritt (a);
(c) Rühren der Mischung von Schritt (b) bis zur Lösung aller Komponenten;
(d) Behandeln bei 70°C für 2 Stunden mit einem Thermostat, bis sich Latexpartikel bilden; und
(e) Separieren der Latexpartikel von Schritt (d);
(f) Sensibilisieren der Latexpartikel mit mono- oder polyklonalen Antikörpern, insbesondere IgG2a, IgG2b, IgG3, IgG1 und IgG2a-k, in einem 0,01 M Glyzinpuffer mit pH 7,0 bis 8,8 bis zur kompletten Bindung;
(g) eine erste Inkubation des Produkts von Schritt (f) bei 38-40°C für nicht mehr als 50-70 Minuten;
(h) eine zweite Inkubation des Produkts von Schritt (f) bei 36-38°C für nicht mehr als 50-70 Minuten;
(i) eine dritte (finale) Inkubation bei 4-8°C für 2-4 Stunden unter Erhalt des Diagnostikums.

2. Verfahren gemäß Anspruch 1, wobei der pH des Glyzinpuffers 7,0 bis 8,8 beträgt, die Dauer und Temperatur der ersten Inkubation bei ungefähr 60 Minuten und ungefähr 39°C liegen, die Dauer und Temperatur der zweiten Inkubation bei ungefähr 60 Minuten und ungefähr 37°C liegen und die Dauer und Temperatur der dritten Inkubation bei ungefähr 3 Stunden und ungefähr 5°C liegen.

3. Verfahren gemäß Anspruch 1, wobei der pH des Glyzinpuffers ungefähr 8,6 beträgt, die Temperatur der ersten Inkubation bei ungefähr 38-40°C liegt, die Dauer bei ungefähr 60 Minuten liegt; die Temperatur und Dauer der zweiten Inkubation bei ungefähr 60 Minuten und ungefähr 37°C liegen; und die Dauer und Temperatur der dritten Inkubation bei ungefähr 3 Stunden und ungefähr 5°C liegen.

4. Verfahren gemäß Anspruch 1, wobei der pH des Glyzinpuffers ungefähr 8,6 beträgt, die Dauer und Temperatur der ersten Inkubation bei ungefähr 60 Minuten und ungefähr 39°C liegen, die Dauer und Temperatur der zweiten Inkubation bei ungefähr 60 Minuten und ungefähr 37°C liegen; und die Dauer und Temperatur der dritten Inkubation bei ungefähr 3 Stunden und ungefähr 5°C liegen.

5. Verfahren gemäß Anspruch 1, wobei der pH des Glyzinpuffers ungefähr 8,6 beträgt, die Dauer und Temperatur der ersten Inkubation bei ungefähr 60 Minuten und ungefähr 38,5°C liegen, die Dauer und Temperatur der zweiten Inkubation bei ungefähr 60 Minuten und ungefähr 36-38°C liegen; und die Dauer und Temperatur der dritten Inkubation bei ungefähr 3 Stunden und ungefähr 5°C liegen.

6. Verfahren gemäß Anspruch 1, wobei der pH des Glyzinpuffers ungefähr 8,6 beträgt, die Dauer und Temperatur der ersten Inkubation bei ungefähr 60 Minuten und ungefähr 38,5°C liegen, die Dauer und Temperatur der zweiten Inkubation bei ungefähr 60 Minuten und ungefähr 37°C liegen; und die Dauer und Temperatur der dritten Inkubation bei ungefähr 3 Stunden und ungefähr 5°C liegen.

7. Verfahren gemäß Anspruch 1, wobei der pH des Glyzinpuffers ungefähr 8,6 beträgt, die Dauer und Temperatur der ersten Inkubation bei ungefähr 60 Minuten und ungefähr 38,5°C liegen, die Dauer und Temperatur der zweiten Inkubation bei ungefähr 60 Minuten und ungefähr 37°C liegen; und die Dauer und Temperatur der dritten Inkubation bei ungefähr 3 Stunden und ungefähr 4-8°C liegen.

8. Verfahren gemäß Anspruch 1, wobei der pH des Glyzinpuffers ungefähr 8,6 beträgt, die Dauer und Temperatur der ersten Inkubation bei ungefähr 60 Minuten und ungefähr 38,5°C liegen, die Dauer und Temperatur der zweiten Inkubation bei ungefähr 60 Minuten und ungefähr 37°C liegen; und die Dauer und Temperatur der dritten Inkubation bei ungefähr 2 bis 4 Stunden und ungefähr 5°C liegen.

9. Diagnostikum zur Bestimmung der Präsenz des gesamten LPS von gramnegativen Bakterien und auch des Genus und Typs von Endotoxin-produzierenden gramnegativen Bakterien, erhältlich durch das Verfahren gemäß einem der Ansprüche 1 bis 8, mit einer Empfindlichkeit von bis zu 4 pg/ml und umfassend:
(a) einen Träger in der Form eines Polystyren-Latex, der mit mono- oder polyklonalen Antikörpern sensibilisiert ist, wobei der Polystyren-Latex-Träger durch Kern-Schale-artige Latexpartikel repräsentiert wird, in welchen der Kern der Polystyren-Latex ist und die Schale der Styren- und Zink-Methacrylat-Kopolymer-Latex ist mit einem Masseneinheitsverhältnis, das von 1:0,8 bis 1:0,4 reicht;
(b) mono- oder polyklonale Antikörper, insbesondere von einer IgG2a und IgG2b oder IgG3 und IgG2a oder IgG1 und IgG2a-k oder IgG1 und IgG2a Subklasse, mit welchen die Latexpartikel sensibilisiert werden.

10. Kit zur Bestimmung der Präsenz von gramnegativen Bakterien, des gesamten LPS von gramnegativen Bakterien und auch des LPS, das für den Genus und Typ von Endotoxin-produzierenden gramnegativen Bakterien gewöhnlich ist, in einem biologischen Material, umfassend das Diagnostikum gemäß Anspruch 9, hergestellt durch ein Verfahren gemäß Anspruch 1, wobei das Kit folgendes umfasst:
(a) einen Behälter, in dem ein Diagnostikum untergebracht ist, welches einen Träger umfasst, der mit mono- oder polyklonalen Antikörpern sensibilisiert ist, insbesondere von einer IgG2a und IgG2b Subklasse, zur Bestimmung der Präsenz von LPS, das allen gramnegativen Bakterien gemein ist;
(b) einen Behälter, in dem ein Diagnostikum untergebracht ist, welches einen Träger umfasst, der mit mono- oder polyklonalen Antikörpern sensibilisiert ist, insbesondere von einer IgG3 und IgG2a Subklasse, zur Bestimmung von LPS, das dem Bakteriengenus gemein ist, insbesondere dem Genus Serratia;
(c) einen Behälter, in dem ein Diagnostikum untergebracht ist, welches einen Träger umfasst, der mit mono- oder polyklonalen Antikörpern sensibilisiert ist, insbesondere von einer IgG1 und IgG2a-k Subklasse, zur Bestimmung von LPS, das dem Bakteriengenus gemein ist, insbesondere dem Genus Klebsiella;
(d) einen Behälter, in dem ein Diagnostikum untergebracht ist, welches einen Träger umfasst, der mit mono- oder polyklonalen Antikörpern sensibilisiert ist, insbesondere von einer IgG1 und IgG2a Subklasse, zur Bestimmung von LPS, das dem Bakteriengenus gemein ist, insbesondere dem Genus Pseudomonas;
(e) einen Behälter, in dem ein Diagnostikum untergebracht ist, welches einen Träger umfasst, der mit mono- oder polyklonalen Antikörpern sensibilisiert ist, insbesondere von einer IgG1 und IgG2a Subklasse, zur Bestimmung von LPS, das dem Bakteriengenus gemein ist, insbesondere dem Genus Enterobacter;
(f) einen Behälter, in dem ein Diagnostikum untergebracht ist, welches einen Träger umfasst, der mit mono- oder polyklonalen Antikörpern sensibilisiert ist, insbesondere von einer IgG2a, IgG2b, IgG3, IgG1 und IgG2a-k Subklasse für verschiedene Typen von gramnegativen Bakterien, insbesondere Serratia, Klebsiella, Pseudomonas und Enterobacter;
(g) eine Packung und Anleitung zur Verwendung.

## Revendications

1. Un procédé de fabrication d'un agent de diagnostic pour déterminer la présence du LPS total de bactéries gram-négatives et aussi du genre et type de bactéries gram-négatives produisant des endotoxines comprenant les étapes de:
(a) fourniture d'un latex de type noyau-enveloppe en mélangeant 10% de styrène, 0,1% de persulfate de potassium, et 89,9% d'eau; le mélange est thermorégulé et agité à 70°C pendant 7 heures jusqu'à conversion complète du monomère;
(b) addition au latex de l'étape (a) de 0,2% de styrène, 0,1% de méthacrylate de zinc, et 0,05% de persulfate de potassium;
(c) agitation du mélange de l'étape (b) jusqu'à dissolution de tous les composants;
(d) thermorégulation à 70°C pendant 2 heures jusqu'à ce que des particules de latex soient formées; et
(e) séparation des particules de latex de l'étape (d);
(f) sensibilisation des particules de latex avec des anticorps mono- ou polyclonaux, en particulier IgG2a, IgG2b, IgG3, IgG1, et IgG2a-k dans un tampon de glycine 0,01 M de pH 7,0 à 8,0 jusqu'à liaison complète;
(g) une première incubation du produit de l'étape (f) à 38-40°C pour pas plus de 50-70 minutes;
(h) une deuxième incubation du produit de l'étape (f) à 36-38°C pour pas plus de 50-70 minutes;
(i) une troisième incubation (finale) à 4-8°C pendant 2-4 heures avec l'obtention de l'agent de diagnostic.

2. Un procédé selon la revendication 1, dans lequel le pH du tampon de glycine est de 7,0 à 8,8, la durée et température de la première incubation sont environ 60 minutes et environ 39°C, la durée et température de la seconde incubation sont environ 60 minutes et environ 37°C et la durée et température de la troisième incubation sont environ 3 heures et environ 5°C.

3. Un procédé selon la revendication 1, dans lequel le pH du tampon de glycine est environ 8,6, la température de la première incubation est environ 38-40°C, la durée est environ 60 minutes; la température et durée de la seconde incubation sont environ 60 minutes et environ 37°C et la durée et température de la troisième incubation sont environ 3 heures et environ 5°C.

4. Un procédé selon la revendication 1, dans lequel le pH du tampon de glycine est environ 8,6, la durée et température de la première incubation est environ 60 minutes et environ 39°C, la durée et température de la seconde incubation sont environ 60 minutes et environ 37°C; et la durée et température de la troisième incubation sont environ 3 heures et environ 5°C.

5. Un procédé selon la revendication 1, dans lequel le pH du tampon de glycine est environ 8,6, la durée et température de la première incubation sont environ 60 minutes et environ 38,5°C, la durée et température de la seconde incubation sont environ 60 minutes et environ 36-38°C; et la durée et température de la troisième incubation sont environ 3 heures et environ 5°C.

6. Un procédé selon la revendication 1, dans lequel le pH du tampon de glycine est environ 8,6, la durée et température de la première incubation sont environ 60 minutes et environ 38,5°C, la durée et température de la seconde incubation sont environ 60 minutes et environ 37°C; et la durée et température de la troisième incubation sont environ 3 heures et environ 5°C.

7. Un procédé selon la revendication 1, dans lequel le pH du tampon de glycine est environ 8,6, la durée et température de la première incubation sont environ 60 minutes et environ 38,5°C, la durée et température de la seconde incubation sont environ 60 minutes et environ 37°C; et la durée et température de la troisième incubation sont environ 3 heures et environ 4-8°C.

8. Un procédé selon la revendication 1, dans lequel le pH du tampon de glycine est environ 8,6, la durée et température de la première incubation sont environ 60 minutes et environ 38,5°C, la durée et température de la seconde incubation sont environ 60 minutes et environ 37°C; et la durée et température de la troisième incubation sont environ 2 à 4 heures et environ 5°C.

9. Un agent de diagnostic pour déterminer la présence du LPS total de bactéries gram-négatives et aussi le genre et type de bactéries gram-négatives produisant des endotoxines qui peut être obtenu par le procédé selon l'une quelconque des revendications 1-8, ayant une sensibilité de jusqu'à 4 pg/ml et comprenant:
(a) un support sous la forme d'un latex de polystyrène avec des anticorps mono- ou polyclonaux sensibilisés sur celui-ci, ledit support de latex de polystyrène étant représenté par des particules de latex de type noyau-enveloppe, dans lesquelles le noyau est le latex de polystyrène et l'enveloppe est le latex de copolymère de méthacrylate de zinc et de styrène dans un rapport d'unité de masse allant de 1:0,8 à 1:0,4;
(b) des anticorps mono- ou polyclonaux, en particulier d'une sous-classe de IgG2a et IgG2b ou IgG3 et IgG2a ou IgG1 et IgG2a-k ou IgG1 et IgG2a, avec lesquels les particules de latex sont sensibilisées.

10. Un kit pour déterminer la présence de bactéries gram-négatives dans un matériel biologique, le LPS total de bactéries gram-négatives, et aussi le LPS général pour le genre et type de bactéries gram-négatives produisant des endotoxines comprenant l'agent de diagnostic selon la revendication 9 produit par un procédé selon la revendication 1, qui comprend:
(a) un récipient, dans lequel un agent de diagnostic est placé comprenant un support sensibilisé avec des anticorps mono- ou polyclonaux, en particulier d'une sous-classe IgG2a et IgG2b pour déterminer la présence de LPS commun à toutes les bactéries gram-négatives;
(b) un récipient, dans lequel un agent de diagnostic est placé comprenant un support sensibilisé avec des anticorps mono- ou polyclonaux, en particulier d'une sous-classe de IgG3 et IgG2a pour déterminer LPS commun pour le genre de bactérie, en particulier le genre Serratia;
(c) un récipient, dans lequel un agent de diagnostic est placé comprenant un support sensibilisé avec des anticorps mono- ou polyclonaux, en particulier d'une sous-classe IgG1 et IgG2a-k pour déterminer LPS commun pour le genre de bactérie, en particulier le genre Klebsiella;
(d) un récipient, dans lequel un agent de diagnostic est placé comprenant un support sensibilisé avec des anticorps mono- ou polyclonaux, en particulier d'une sous-classe IgG1 et IgG2a pour déterminer LPS commun pour le genre de bactérie, en particulier le genre Pseudomonas;
(e) un récipient, dans lequel un agent de diagnostic est placé comprenant un support sensibilisé avec des anticorps mono- ou polyclonaux, en particulier d'une sous-classe IgG1 et IgG2a pour déterminer LPS commun pour le genre de bactérie, en particulier le genre Enterobacter;
(f) un récipient, dans lequel un agent de diagnostic est placé comprenant un support sensibilisé avec des anticorps mono- ou polyclonaux, en particulier d'une sous-classe de IgG2a, IgG2b, IgG3, IgG1, et IgG2a-k pour des types variés de bactéries gram-négatives, en particulier, Serratia, Klebsiella, Pseudomonas, et Enterobacter.
(g) un paquet et des instructions pour l'emploi.
